(19)

Europäisches
Patentamt
European
Patent Office
Office européen
des brevets

(11) **EP 2 019 665 B1**

(12) **EUROPEAN PATENT SPECIFICATION**

(45) Date of publication and mention
of the grant of the patent:
**19.09.2018   Bulletin 2018/38**

(21) Application number: **07718849.8**

(22) Date of filing: **04.05.2007**

(51) Int Cl.:
*A61K 9/107* *(2006.01)*        *A61K 9/22* *(2006.01)*
*A61K 9/54* *(2006.01)*

(86) International application number:
**PCT/AU2007/000602**

(87) International publication number:
**WO 2007/128066 (15.11.2007 Gazette 2007/46)**

(54) **DRUG RELEASE FROM NANOPARTICLE-COATED CAPSULES**

ARZNEIFREISETZUNG VON MIT NANOTEILCHEN ÜBERZOGENEN KAPSELN

LIBÉRATION DE MÉDICAMENTS À PARTIR DE GÉLULES RECOUVERTES DE NANOPARTICULES

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **04.05.2006  AU 2006902311
07.12.2006  AU 2006906840**

(43) Date of publication of application:
**04.02.2009   Bulletin 2009/06**

(73) Proprietor: **Reformpharm Pty Ltd
Wayville, S.A. 5034 (AU)**

(72) Inventors:
• **PRESTIDGE, Clive, Allan
Semaphore South, S.A. 5019 (AU)**
• **ESKANDAR, Nasrin, Ghouchi
Glynde, S.A. 5070 (AU)**
• **SIMOVIC, Spomenka
Mawson Lakes, S.A. 5095 (AU)**

(74) Representative: **Gill Jennings & Every LLP
The Broadgate Tower
20 Primrose Street
London EC2A 2ES (GB)**

(56) References cited:
EP-A1- 0 478 326        WO-A1-2006/130904
WO-A1-2006/133518       WO-A1-2007/041627
WO-A2-02/47665          WO-A2-2007/115134
US-A1- 2004 202 682     US-B1- 6 585 983

• HWANG Y J ET AL: "Controlled release of retinol
from silica particles prepared in O/W/O emulsion:
The effects of surfactants and polymers",
JOURNAL OF CONTROLLED RELEASE,
ELSEVIER, AMSTERDAM, NL, vol. 106, no. 3, 2
September 2005 (2005-09-02), pages 339-349,
XP027664175, ISSN: 0168-3659 [retrieved on
2005-09-02]
• NOBLE P.F. ET AL.: 'Fabrication of "Hairy"
Colloidosomes with Shells of Polymeric
Microrods' JOURNAL AM. CHEM. SOC.
COMMUNICATIONS vol. 126, 2004, pages 8092 -
8093, XP002355521
• DINSMORE A.D. ET AL.: 'Colloidosomers:
Selectively Permeable Capsules Composed of
Colloidal Particles' SCIENCE vol. 298, 01
November 2002, pages 1006 - 1009, XP002229136
• HWANG Y.J. ET AL.: 'Controlled Release of
Retinol from Silica Particles Prepared in O/W/O
emulsion: The Effects of Surfactants and
Polymers' JOURNAL OF CONTROLLED
RELEASE vol. 106, 2005, pages 339 - 349,
XP005038454

EP 2 019 665 B1

## Description

### FIELD OF THE INVENTION

The present invention relates to the encapsulation of a material within particles and, in particular, the encapsulation by nanoparticles of a liquid droplet or a lipid vesicle (i.e. liposomes), which may comprise an active substance.

### BACKGROUND OF THE INVENTION

The development of new forms of active substances such as drug compounds and pesticides, as well as a desire to increase the efficacy of existing substances, has created a need to develop new and effective ways of delivering substances to their appropriate targets. For example, it is likely that many potentially useful active substances have not been commercialised because of inadequate formulation. In many cases, the inability to formulate the active substance into a deliverable form could simply be due to solubility problems.

Emulsions and liposomes are vehicles which may be used to carry active substances such as drug compounds, cosmetics, pesticides, foodstuffs and nutriceuticals, etc., to target areas. Emulsions are dispersed systems consisting of two immiscible liquids, one of which is dispersed (the dispersed or discontinuous phase) in the continuous phase, as droplets. The dispersed droplets may comprise or include a suitably soluble substance, e.g. an active substance such as a drug compound or a pesticide; the dispersed droplets thereby acting as delivery vehicles. If the emulsified droplets are oil droplets, then the emulsion can solubilise or complex amphiphilic or lipophilic active substances, whereas, if the emulsified droplets are aqueous, then water-soluble active substances can be entrapped.

Liposomes or vesicles are another type of delivery vehicle, consisting of bi-layered structures that are commonly built up using phospholipids, with one or several bilayers of phospholipids surrounding an aqueous liquid core. Most pharmaceutical research with liposomes has focused on water-soluble drug compounds entrapped in the aqueous liquid core.

Although useful as vehicles for the delivery of active substances, most emulsions and liposomes are limited by the fact they are thermodynamically unstable and, generally, over time, will coalesce and may eventually separate into two distinct liquid phases (emulsions) or otherwise degrade and release the liquid core into the surrounding media (liposomes). This instability is exacerbated in veterinary and pharmaceutical applications since the vehicles are used under circumstances (e.g. increased salt (electrolyte) or variations in pH) which may put a severe strain on the vehicle structure. The degradation of vehicles containing active substances is undesirable since considerable time and effort is spent in formulating the delivery system. In the veterinary, pharmaceutical and nutriceutical industries in particular, if vehicle stability is compromised, the bioavailability of the active substance may be affected.

Encapsulation technology is generally directed to encapsulating core materials in a protective coating until time of use. The core material can be a liquid such as oil or water or it can be a solid or a crystal. The encapsulation of a liquid usually facilitates the dispersion of the encapsulated liquid core in another liquid. Encapsulated droplets of oils or water are particularly useful in industries where the delivery of, and/or protection of, active substances is required, for example, the cosmetics and pharmaceutical industries, etc. Active substances that are insoluble in aqueous media, such as drug compounds, can be encapsulated in a liquid in which it will dissolve. The capsule can then be dispersed in a medium (such as body fluid) in which it may not otherwise have been compatible.

Particle stabilisation of liposomes is not well-known. A problem with known particle-stabilised emulsions (capsules) is that the stability of the capsules remains poor over a period of time. This means that it is difficult to transport the capsules over long distances and it is difficult to store the capsules for a delayed time of use. As the capsules degrade, the active substance (e.g. a drug compound or a pesticide) within the capsules can leach out, or may be released without control. This leaching or uncontrolled release can pose a more serious problem when aged capsules are used, for example, in the delivery of certain drugs in the body, since one aim of the encapsulation process is to shield healthy cells from the drug's toxicity and prevent the drug from concentrating in vulnerable tissues (e.g. the kidneys and liver).

Existing preparations of particle-stabilised vehicles (capsules) are usually dispersed in a liquid in order that the capsules can be delivered to the body as a liquid suspension. These liquid formulations usually have a low active substance content to liquid ratio and, in addition, there is a risk of microbial growth in the liquid which can cause serious infections or spoilage.

A further problem is coalescence of the capsules to form capsules with an increased diameter. Larger capsules are less stable over time, and larger capsules cannot be delivered to some areas where the diameter of the capsule will not be permitted, e.g. capillaries in the body. Further to this, active substance release profiles are correlated with interfacial surface area. It is important, therefore, that capsule size remain constant in order that the release profile of the active substance is maintained.

WO2006/130904, which was filed before but published after the present application was filed (Article 54(3) EPC), describes a method of producing a dried formulation for an active substance comprising dispersing a discontinuous phase (e.g. an oil based or lipidic medium) comprising the active substance into a continuous phase (e.g. water) forming droplets of the discontinuous phase, allowing nanoparticles to congregate and coat the surface of the droplets and thereafter removing the continuous phase from the nanoparticle coated droplets to produce a dried formulation.

WO2007/115134, which was filed before but published after the present application was filed (Article 54(3) EPC), describes formulations of neutral retinoids, in particular fenretinide (HPR) in the form of lipid nanoparticles, solid dispersions and emulsions.

Accordingly, there is required an improved vehicle for the delivery and/or storage of an active compound which eliminates at least some of the problems associated with the delivery systems discussed above.

It is an object of the present invention to provide an encapsulated droplet which is relatively stable against leaching and coalescence, and which allows for the release of an active substance in a controlled manner.

## SUMMARY OF THE INVENTION

The present invention provides a method of producing a controlled release formulation for an active substance, said method comprising the steps of:

(i) dispersing a discontinuous phase comprising 0.01 to 10wt% an active substance into a continuous phase in the presence of an emulsifier with an HLB value of less than 12 so as to form a two-phase liquid system comprising droplets of said discontinuous phase, each of said droplets having, at its surface, a phase interface; and

(ii) allowing hydrophilic silica nanoparticles, provided to said two-phase liquid system to congregate at the phase interface to thereby coat said surface of the droplets in at least one layer of said nanoparticles, and wherein the ratio of nanoparticle size to the size of the nanoparticle-coated droplets does not exceed 1:15;

wherein said two-phase liquid system is formed, or is otherwise adjusted, so as to have a concentration of sodium chloride (NaCl) electrolyte which controls the nanoparticle congregation of step (ii) by enhancing nanoparticle congregation such that the coating on said surface of the droplets provided by the at least one layer of said nanoparticles, presents a semi-permeable barrier to the active substance, thereby controlling release of the active substance.

Preferably, the concentration of NaCl is within the range of $5 \times 10^{-4}$ to $5 \times 10^{-1}$ M preferably within the range of $1 \times 10^{-3}$ to $1 \times 10^{-1}$ M.

Preferably, the discontinuous phase is an oil-based or lipidic medium and the continuous phase is aqueous.

Preferably, the discontinuous phase is aqueous and each droplet is surrounded by a single or multiple lipid bilayer to form a liposome, and the continuous phase is also aqueous.

The active substance may be selected from nutriceutical substances, cosmetic substances (including sunscreens), pesticide compounds, agrochemicals and foodstuffs. However, preferably, the active substance is selected from drug compounds and vitamins.

Where the discontinuous phase is an oil-based or lipidic medium, the present invention is particularly suited to the production of a controlled release formulation for lipophilic drug compounds (in otherwise, poorly soluble drugs).

Preferably, said nanoparticles have an average diameter of 5 - 2000 nm, more preferably, 20 - 80 nm, most preferably about 50 nm.

Preferably, the nanoparticles are provided to the two-phase liquid system by inclusion in the discontinuous phase.

An emulsifier is used to stabilise the emulsion prior to the congregation of the nanoparticles. Suitable emulsifiers include lecithin, oleylamine, sodium deoxycholate, 1,2-distearyl-sn-glycero-3-phosphatidyl ethanolamine-*N*, stearylamine, amino acids (e.g. lysine, phenylalanine or glutamic acid) and 1,2-dioleoyl-3-trimethylammonium-propane. Hydrophilic emulsifiers such as sodium dodecyl sulphate (SDS) are less suitable, since these can readily migrate into the continuous phase where they can coat both the droplets and the nanoparticles, when present in high concentrations, thereby preventing nanoparticle congregation.

Preferred emulsifiers are lecithin (which confers a negative charge to the droplets) and oleylamine (which confers a positive charge to the droplets).

The emulsifier will typically be provided in an amount in the range of 0.0001 to 100 wt% of the emulsion, more preferably, in the range of 0.005 to 50 wt%, and most preferably, in the range of 0.01 to 1 wt% of the emulsion.

The two-phase liquid system is formed, or is otherwise adjusted, so as to have a concentration of the electrolyte which enhances the nanoparticle congregation of step (ii) such that the coating on said surface of the droplets (i.e. the coating provided by the at least one layer of said nanoparticles), presents a semi-permeable barrier to the active substance. By "semi-permeable barrier", it is to be understood that the coating substantially retards the diffusion of the active substance from within the encapsulated droplets, such that the active substance is released in a controlled manner, in particular, in a sustained manner. Preferably, the semi-permeable barrier presented by the nanoparticle coating retards the diffusion of the active substance from within the encapsulated droplets such that after two hours of being placed in a test medium (e.g. MilliQ water), at least 25% of the active substance content of the encapsulated droplets has been retained within the encapsulated droplets (ie no more than 75% of the active substance content has been released into the test medium). More preferably, the semi-permeable barrier retards the diffusion of the active substance content of the encapsulated droplets such that at least 35%, and most preferably at least 45%, of the active substance has been retained within the encapsulated droplets after two hours of being placed in a test medium.

The two-phase liquid system may be adjusted, prior to step (ii), so as to have a concentration of the electrolyte which enhances the nanoparticle congregation of step (ii) by adding or removing an amount of the said suitable electrolyte. However, conveniently, the two-phase liquid system is formed so as to have the required concentration of the suitable electrolyte. For example, the two-phase liquid system can be formed by dispersing a discontinuous phase into a continuous phase which comprises said concentration of the suitable electrolyte.

The said concentration of NaCl electrolyte will typically be at least $5 \times 10^{-4}$ M, preferably, at least $10^{-3}$ M. More preferably, the concentration of electrolyte is within the range of $5 \times 10^{-4}$ to $5 \times 10^{-1}$ M. Most preferably, the concentration of the electrolyte will be in the range of $1 \times 10^{-3}$ to $1 \times 10^{-1}$ M. A lesser concentration of electrolyte may, however, suffice (e.g. $1 \times 10^{-6}$ to $1 \times 10^{-5}$ M).

Optionally, the encapsulated droplets are provided with a polymer layer around the periphery to modify the interfacial properties of the capsule.

Preferably, the method of the first aspect further comprises a drying step (iii) to produce a dried formulation. The drying step may be performed using a rotary evaporator. Alternatively, the drying step may be performed by freeze drying, spray drying, fluidised bed procedures or pressure filtration combined with vacuum drying. The encapsulated droplets (i.e. capsules) of the dried formulation can be readily re-dispersed into a liquid (preferably, water or aqueous solution) to re-form a two-phase liquid system, thereby providing a useful formulation for the controlled release of an active substance such as a drug compound.

The discontinuous phase may, optionally, be cross-linked or otherwise comprise a gelling material so as to form a matrix. Such a matrix may enhance the controlled release (i.e. sustained release) of an active substance from the encapsulated droplets.

Also described herein is a method of producing a controlled release formulation for an active substance, said method comprising the steps of:

(i) dispersing a discontinuous phase comprising an active substance into a continuous phase so as to form a two-phase liquid system comprising droplets of said discontinuous phase, each of said droplets having, at its surface, a phase interface; and

(ii) allowing nanoparticles provided to said two-phase liquid system to congregate at the phase interface to thereby coat said surface of the droplets in at least one layer of said nanoparticles;

wherein the active substance is present in the discontinuous phase in an amount greater than its solubility limit in the discontinuous phase.

Preferably, the discontinuous phase is an oil-based or lipidic medium and the continuous phase is aqueous. Alternatively, the discontinuous phase is aqueous.

Further, the discontinuous phase is aqueous and each droplet surrounded by a single or multiple lipid bi-layer to form a liposome, and the continuous phase is also aqueous.

Again, the active substance may be selected from nutriceutical substances, cosmetic substances, pesticide compounds, agrochemicals and foodstuffs. However, preferably, the active substance is selected from drug compounds (and, particularly, lipophilic drug compounds where the discontinuous phase used is an oil-based or lipidic medium).

In the described method, the active substance is necessarily present in an amount that is greater than its solubility limit in the discontinuous phase. Preferably, that amount will be at least about 110%, more preferably at least about 120%, of the solubility limit of the active substance in the discontinuous phase. However, amounts that provide a discontinuous phase supersaturated with the active substance are also suitable. Such amounts may be up to about 300% or more of the solubility limit of the active substance in the discontinuous phase. Such amounts can be achieved by, for example, increasing the solubility of active substance in the discontinuous phase by the presence of an emulsifier or by otherwise initially providing the nanoparticles in the discontinuous phase.

The nanoparticles may be hydrophilic or hydrophobic. Preferably, said nanoparticles have an average diameter of 5 - 2000 nm, more preferably, 20 - 80 nm, most preferably, about 50 nm. Also, preferably, the size of the nanoparticles will be such that the ratio of nanoparticle size to capsule size (i.e. the size of the encapsulated droplets) does not exceed 1:15. Moreover, nanoparticles for use in the method of the second aspect, are preferably silica nanoparticles.

Optionally, an emulsifier (e.g. lecithin or oleylamine) or amino acids (e.g. lysine, phenylalanine or glutamic acid) can be used to stabilise the emulsion prior to the congregation of the nanoparticles.

The coating formed on said surface of the droplets (i.e. the coating provided by the at least one layer of said nanoparticles), presents either a permeable or semi-permeable barrier to the active substance. With hydrophilic nanoparticles, a suitable permeable coating may be achieved by forming, or otherwise adjusting, the two-phase liquid system so as to have a concentration of a suitable electrolyte (e.g. NaCl) in the range of $5 \times 10^{-3}$ to $1 \times 10^{-1}$ M, more preferably about $1 \times 10^{-2}$ M. With hydrophilic nanoparticles, a suitable permeable coating may be achieved by forming, or otherwise adjusting, the two-phase liquid system so as to have a concentration of a suitable electrolyte in the range of $5 \times 10^{-5}$ to $5 \times 10^{-3}$ M, more preferably about $1 \times 10^{-4}$ M.

Optionally, the encapsulated droplets are provided with a polymer layer around the periphery to modify the interfacial properties of the capsule.

Preferably, the method further comprises a drying step (iii) to produce a dried formulation.

In a further disclosure a formulation produced in accordance with the disclosed methods is provided.

Preferably, the formulation is a dried formulation.

The formulation may be suitable for use in a range of dosage forms including oral dosage forms (e.g. tablets, caplets, capsules, liquid emulsions and suspensions and elixirs), mucosal dosage forms, nasal dosage forms (e.g. sprays and aerosols) and topical dosage forms (e.g. creams and lotions).

The present disclosure also provides a controlled release formulation for topical application to the skin (i.e. epidermis including the stratum corneum, and dermis), wherein said formulation comprises droplets of an oil-based or lipidic medium comprising retinol (Vitamin A) or a retinol derivative and, optionally, an emulsifier, and wherein said droplets are at least partially coated on their surface with nanoparticles.

Preferably, the formulation comprises droplets of an oil-based medium (e.g. triglyceride oils, Paraffin oils, Soybean oils and Jojoba oils).

Preferably, the formulation comprises retinol, however certain retinol derivatives such as retinyl palmitate and retinyl acetate may also be suitable.

Further, the formulation may comprise active ingredients commonly included in cosmetics such as anti-wrinkle and/or anti-ageing creams, or sunscreens. For example, tocopherols (Vitamin E), coenzyme Q10 (ubiquinone), UV-A absorbers (e.g. avobenzene) and UV-B absorbers (e.g. octyl methoxycinnamate), titanium dioxide and zinc oxide.

Preferably, the droplets of the formulation are coated on their surface with at least one layer of nanoparticles. Partial coatings (e.g. coatings which coverr at least 10%, more preferably at least 50%, of the droplet surfaces, are also suitable.

## BRIEF DESCRIPTION OF THE DRAWINGS

An illustrative embodiment of the present invention will be discussed with reference to the accompanying drawings and examples wherein:

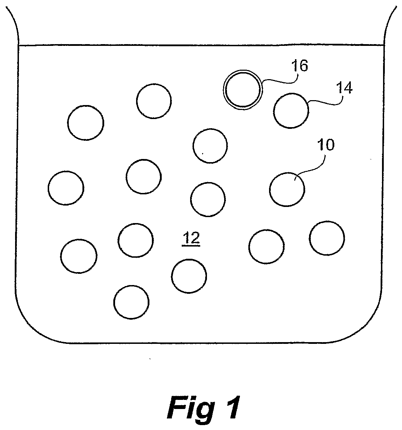

Figure 1 is a cross-sectional schematic of an emulsion known in the art;

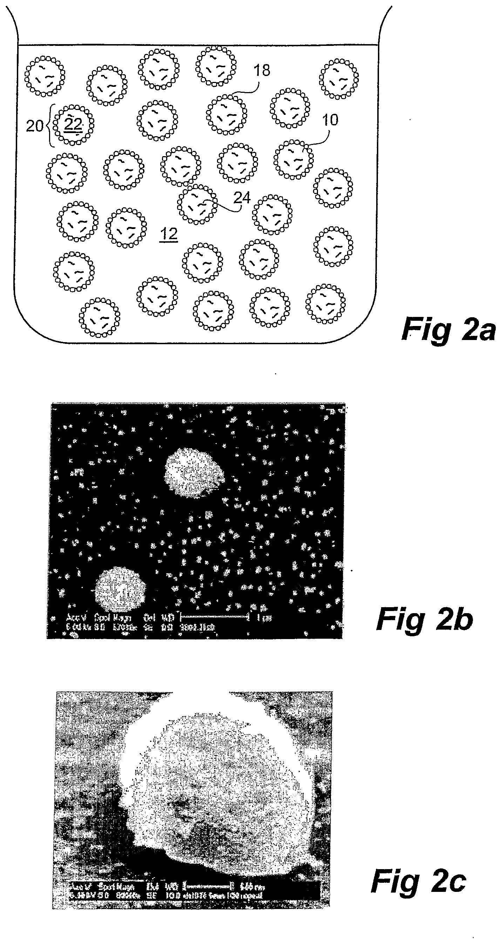

Figure 2 is a cross-sectional schematic of a nanoparticle-stabilised emulsion as described herein;

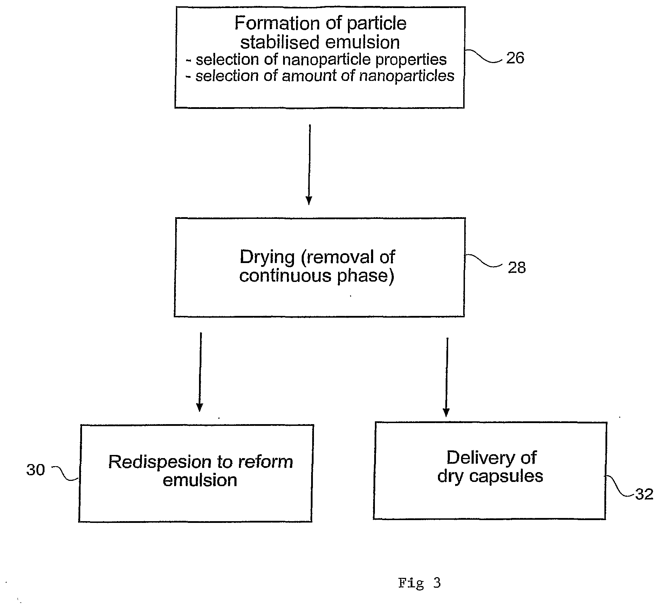

Figure 3 is a flow chart showing the steps involved in obtaining dry capsules of the present invention;

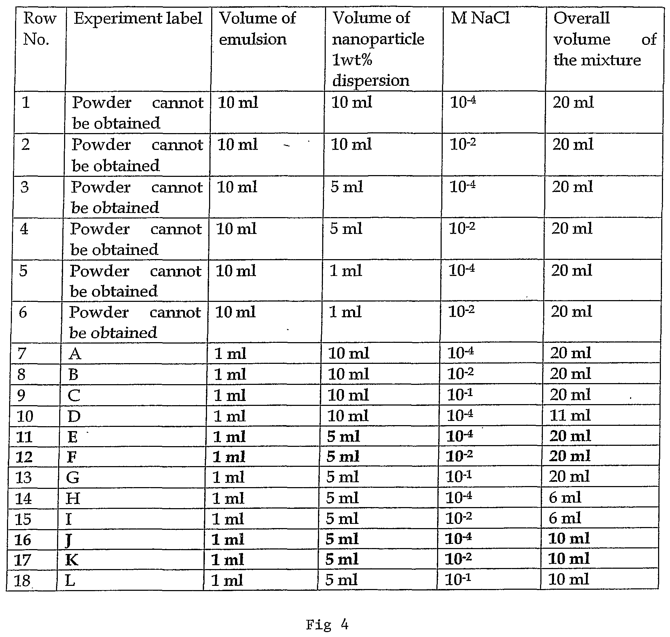

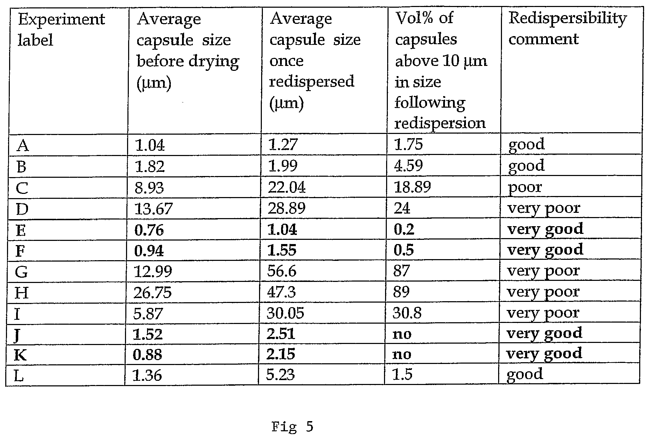

Figure 4 is a table showing the volumes of emulsion and volumes of nanoparticles (1 % wt aqueous dispersion) as well as salt concentrations that produce dry capsules that can be effectively re-dispersed (see Figure 5);

Figure 5 is a table showing the average capsule size following re-dispersion of the capsules listed in the table of Figure 4;

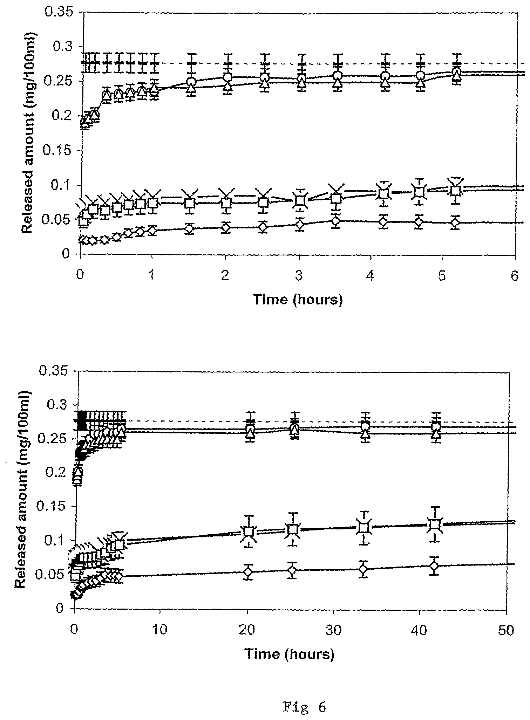

Figure 6 is a graph showing drug release profiles from liquid PDMS emulsion ($\phi$=0.01) containing 0.025 wt% DBP, 900 ml MilliQ (MQ) water as dissolution medium (t=37°C; 50 rpm), where $\bigcirc$ shows the results for bare PDMS droplets and the results for PDMS droplets coated by hydrophobic silica are shown as : $\triangle$ $10^{-4}$ M NaCl; $\square$ $10^{-3}$ M NaCl; $\times 10^{-2}$ M NaCl; $\diamondsuit 10^{-1}$ M NaCl; the dashed line represents the maximal possible DBP concentration in dissolution medium: a) release time first 6 hours; b) release time 50 hours;

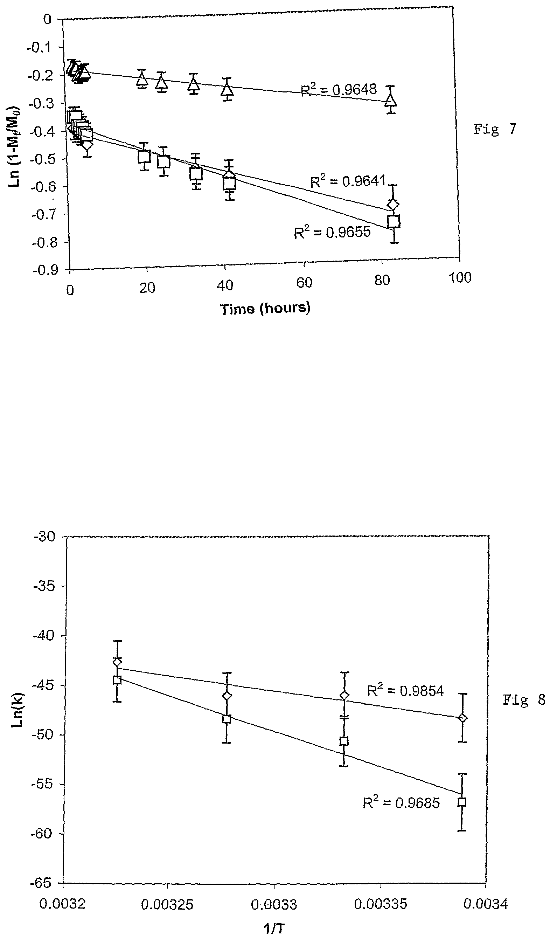

Figure 7 is a graph of Ln ($1-M_t/M_0$) versus time for DBP release profiles from hydrophobic nanoparticle-coated liquid PDMS emulsion ($\phi$=0.01) containing 0.025 wt% DBP, 900 ml MQ water as dissolution medium, where $\square$ $10^{-3}$ M NaCl; $\diamondsuit$ $10^{-2}$ M NaCl; $\triangle 10^{-1}$ M NaCl;

**Figure 8** is a graph showing Arrhenius plots for DBP release profiles from hydrophobic coated liquid PDMS emulsion droplets ($\phi$=0.01) containing 0.025 wt% DBP, 900 ml MQ water as dissolution medium, where □ $10^{-1}$ M NaCl; ◇ $10^{-3}$ M NaCl;

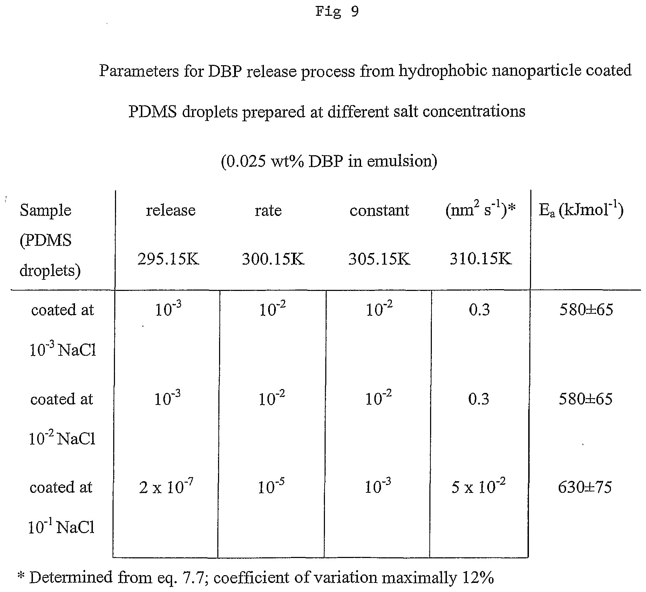

**Figure 9** is a Table showing parameters for DBP release process from hydrophobic nanoparticle-coated PDMS droplets prepared at different salt concentrations (0.025 wt% DBP in emulsion);

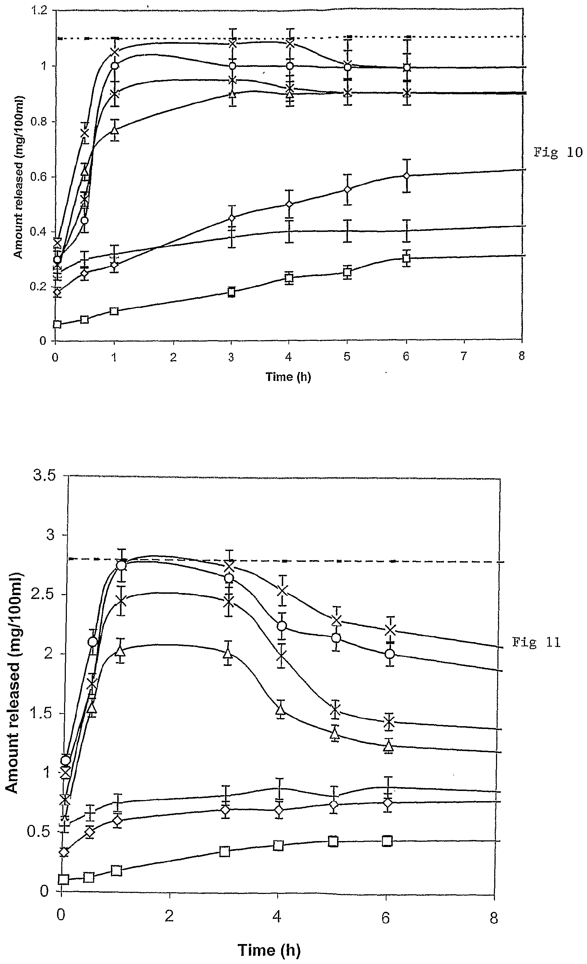

**Figure 10** is a graph showing DBP release profiles in 900 ml MQ water: pure DBP added: 1.1mg/100ml and DBP 0.1 % wt in PDMS emulsion droplets ($\phi$=0.01), where ◇ bare liquid PDMS droplets; □ pure DBP; Δ pure DBP with hydrophilic silica nanoparticles; ○ pure DBP with hydrophobic silica nanoparticles; ∗ PDMS droplets coated by hydrophilic silica ($10^{-2}$ M NaCl); × PDMS droplets coated by hydrophobic silica nanoparticles ($10^{-4}$ M NaCl); + PDMS droplets coated by hydrophobic silica nanoparticles ($10^{-1}$ M NaCl); the dotted line corresponds to the maximal DBP concentration that can be achieved in the dissolution medium;

**Figure 11** is a graph of DBP release profiles in 900 ml MQ water, pure DBP added: 2.8mg/100ml and DBP (0.25% wt) in PDMS emulsion droplets ($\phi$=0.01): ◇ bare liquid PDMS droplets; □ pure DBP; Δ pure DBP with hydrophilic silica nanoparticles; ○ pure DBP with hydrophobic silica nanoparticles; ∗ PDMS droplets coated by hydrophilic silica nanoparticles ($10^{-2}$ M NaCl); × PDMS droplets coated by hydrophobic silica nanoparticles ($10^{-4}$ M NaCl); + PDMS droplets coated by hydrophobic silica nanoparticles (10-1 M NaCl); dotted line correspond to the maximal DBP concentration that can be achieved in the dissolution medium;

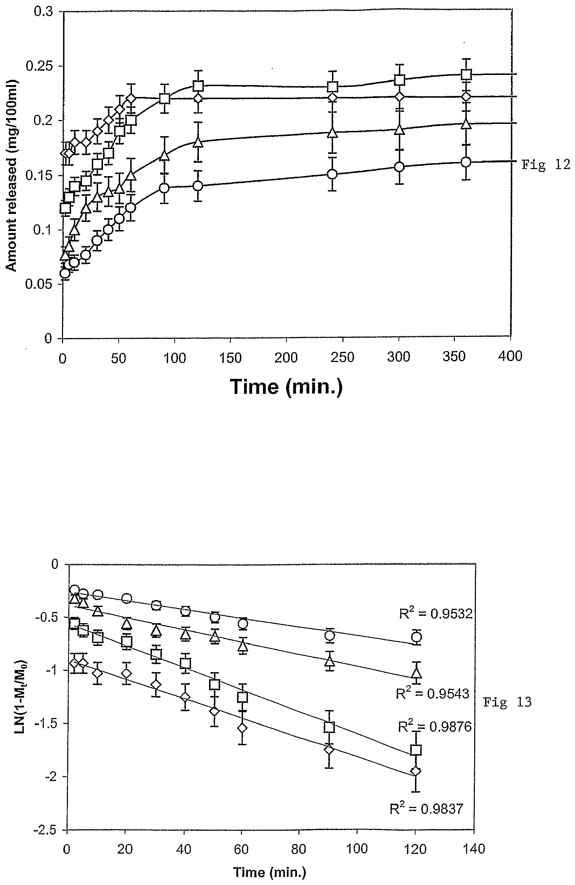

**Figure 12** is a graph showing DBP release profiles from cross-linked PDMS emulsion droplets ($\phi$=0.01) containing 0.025 wt% DBP, 900 ml MQ water as dissolution medium, where ◇ bare PDMS droplets and PDMS droplets coated by hydrophobic silica nanoparticles: □ $10^{-4}$ M NaCl; Δ $10^{-3}$ M NaCl; ○ $10^{-1}$ M NaCl;

**Figure 13** is a graph showing Ln ($1-M_0/M_t$) versus time for drug release profiles from cross-linked PDMS emulsion droplets ($\phi$=0.01) containing 0.025 ml MQ water as dissolution medium, where ◇ bare PDMS droplets and PDMS droplets coated by hydrophobic silica nanoparticles; □ $10^{-4}$ M NaCl; Δ $10^{-3}$ M NaCl; ○ $10^{-1}$ M NaCl;

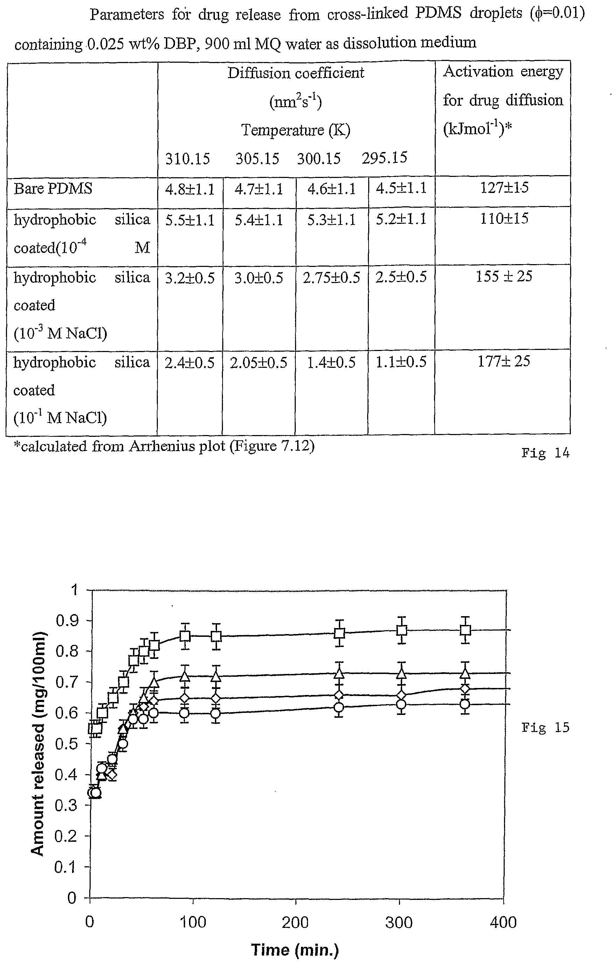

**Figure 14** is a Table showing parameters for drug release from cross-linked PDMS droplets ($\phi$=0.01) containing 0.025 wt% DBP, 900 ml MQ water as dissolution medium;

**Figure 15** is a graph showing DBP release profiles from cross-linked PDMS emulsion droplets ($\phi$=0.01) containing 0.25 wt% DBP in emulsions, 900 ml MQ water as dissolution medium, where ◇ bare PDMS droplets and PDMS droplets coated by hydrophobic silica nanoparticles: □ $10^{-4}$ M NaCl; Δ $10^{-3}$ M NaCl; ○ $10^{-1}$ M NaCl;

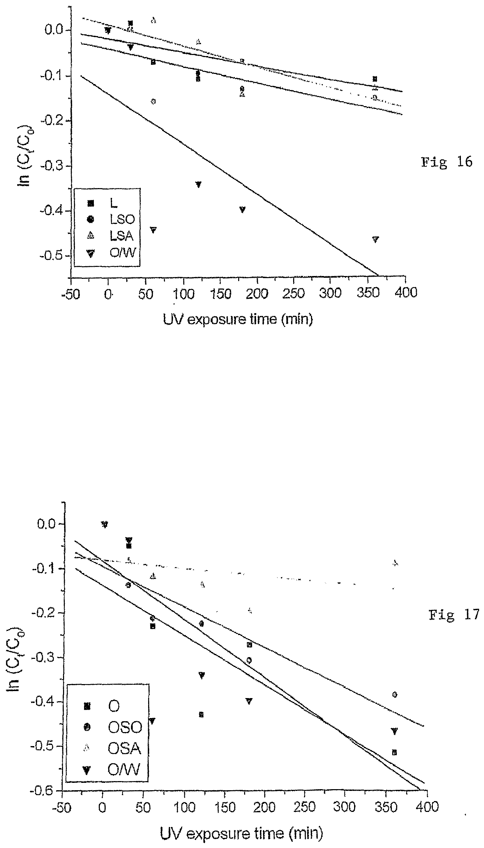

**Figure 16** provides a graph showing the degradation kinetics of retinol contained in negatively charged nanoparticle-coated capsules, wherein the emulsion is stabilised by lecithin (■ lecithin stabilised bare emulsion (L); ● lecithin stabilised emulsion with silica in oil phase (LSO); ▲ lecithin stabilised bare emulsion with silica in water phase (LSA); and ▼ oil in water emulsion (O/W));

**Figure 17** provides a graph showing the degradation kinetics of retinol contained in positively charged nanoparticle-coated capsules, wherein the emulsion is stabilised by oleylamine (■ oleylamine stabilised bare emulsion (O); ● oleylamine stabilised emulsion with silica in oil phase (OSO); ▲ oleylamine stabilised emulsion with silica in water phase (OSA); and ▼ oil in water emulsion (O/W));

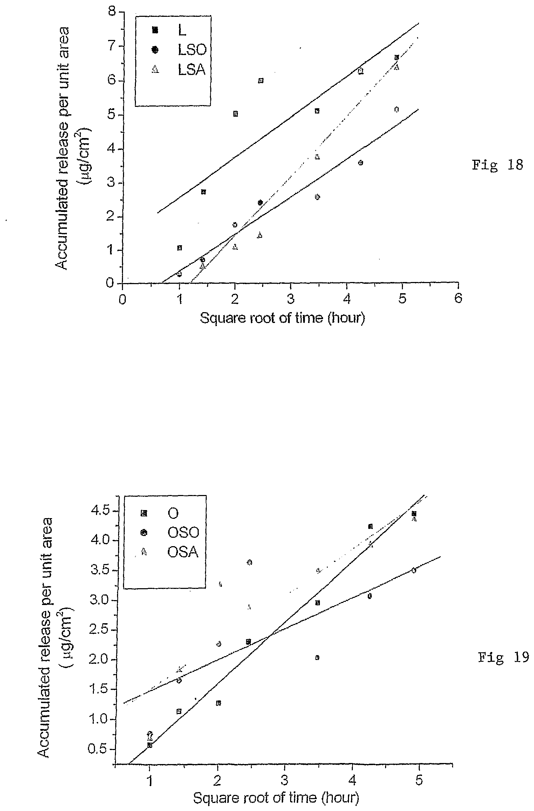

**Figure 18** provides a graph of the release profile of retinol from negatively charged nanoparticle-coated capsules (■ lecithin stabilised bare emulsion (L); ● lecithin stabilised emulsion with silica in oil phase (LSO); and ▲ lecithin stabilised emulsion with silica in water phase (LSA));

**Figure 19** provides a graph of the release profile of retinol from positively charged nanoparticle-coated capsules (■ oleylamine stabilised bare emulsion (O); ● oleylamine stabilised emulsion with silica in oil phase (OSO); ▲ oleylamine stabilised emulsion with silica in water phase (OSA));

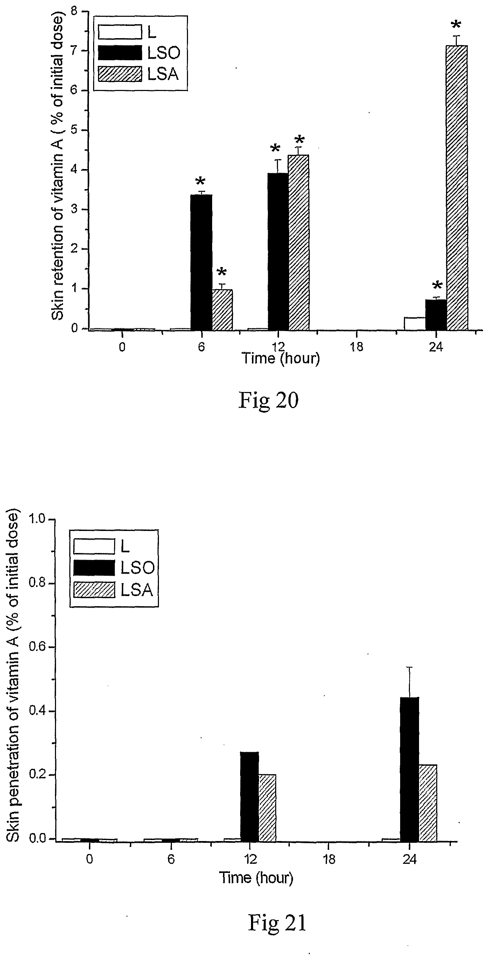

**Figure 20** provides a graph showing the retention of vitamin A (retinol) in pig skin over 24 hours from a lecithin-stabilised formulation of the present invention (L = lecithin-stabilised emulsion of all-*trans*-retinol in a triglyceride oil; LSO = lecithin-stabilised nanoparticle-coated emulsion of all-*trans*-retinol in a triglyceride oil, wherein the capsules were formed from a mix with the nanoparticles in the oil phase; and LSA = lecithin-stabilised nanoparticle-coated emulsion of *all-trans*-retinol in a triglyceride oil, wherein the capsules were formed from a mix with the nanoparticles in the aqueous phase);

**Figure 21** provides a graph showing the penetration of vitamin A (retinol) through pig skin from a lecithin-stabilised formulation of the present invention (L = lecithin-stabilised emulsion of all-*trans*-retinol in a triglyceride oil; LSO = lecithin-stabilised nanoparticle-coated emulsion of all-*trans*-retinol in a triglyceride oil, wherein the capsules were formed from a mix with the nanoparticles in the oil phase; and LSA = lecithin-stabilised nanoparticle-coated emulsion of all-*trans*-retinol in a triglyceride oil, wherein the capsules were formed from a mix with the nanoparticles in the aqueous phase); and

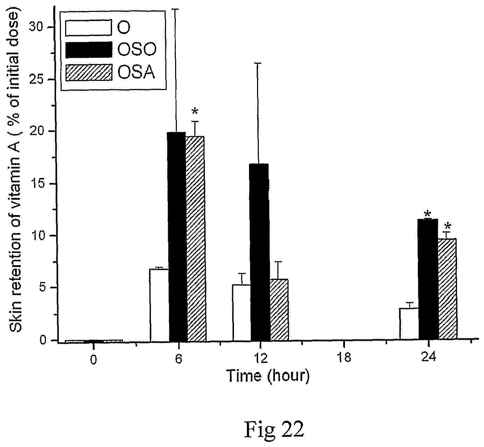

**Figure 22** provides a graph showing the retention of vitamin A (retinol) in pig skin over 24 hours from a oleylamine-stabilised formulation of the present invention (O = oleylamine-stabilised emulsion of all-*trans*-retinol in a triglyceride oil; OSO = oleylamine-stabilised nanoparticle-coated emulsion of all-*trans*-retinol in a triglyceride oil, wherein the capsules were formed from a mix with the nanoparticles in the oil phase; and OSA = oleylamine-stabilised nanoparticle-coated emulsion of all-*trans*-retinol in a triglyceride oil, wherein the capsules were formed from a mix with the nanoparticles in the aqueous phase).

## DESCRIPTION OF PREFERRED EMBODIMENT

Figure 1 is a cross-sectional schematic of an emulsion according to the prior art, showing an immiscible two-phase system having an oil phase in the form of droplets 10 dispersed in a continuous aqueous phase 12. Droplets 10 are dispersed in the continuous phase 12, thereby defining a phase interface 14. Emulsions are thermodynamically unstable and, in general, separate into the component phases over time. After a period of time, adjacent oil droplets 10 will coalesce (the beginning of phase separation) to form larger oil droplets. Phase separation is controlled by both coalescence and Ostwald ripening. The rate of coalescence is determined by the stability against drainage and rupture of the thin film separating two contacting droplets. The rate of Ostwald ripening is controlled by the molecular solubility of the dispersed phase in the continuous phase. If an emulsion is not stabilised by an emulsifier localised in the thin film 16, then these coalescence and ripening processes occur within minutes. Eventually the oil phase 10 and aqueous phase 12 will have completely separated into the two component phases (oil and water).

Figure 2 depicts a cross-sectional schematic of an emulsion formed by mixing oil and water phases with a rotor-stator homogeniser. It will be understood that any method of preparing an emulsion could be employed, for example, high pressure homogenisation. In order to improve biocompatibility of the emulsion, the oil phase can be a fatty-food simulant such as Miglyol 812™. Alternatively, the oil phase can be a silicone such as polydimethlysiloxane (PDMS), or any other oily medium which will form an emulsion with an aqueous phase.

Figure 2 shows the system of Figure 1 where droplets 10 have been stabilised by nanoparticles 18 at the interface 14. Two otherwise immiscible liquids (10 and 12) have thereby formed a stabilised emulsion. Persons skilled in the art will understand that the two-phase system may comprise any two immiscible liquids. It should also be understood that liposomes dispersed within a liquid are also within the scope of the invention. Where an emulsion defines a discontinuous phase of droplets, a liposome is a vehicle dispersed within a continuous phase. The liquid core or discontinuous phase of the liposome is separated from the liquid of the continuous phase by a bi-layered structure of lipids.

Nanoparticles 18 can be dispersed in a liquid by sonication and added to the emulsion. In the preferred embodiment, the liquid dispersion comprises 1 % by weight (1 wt%) of nanoparticles in an aqueous medium. However, other weight % dispersions can be usefully employed. Upon addition, the nanoparticles congregate at the phase interface 14 by, for example, self-assembly. Alternatively, rather then being added to the pre-formed emulsion, nanoparticles 18 can be first dispersed in either phase (oil or water) and, as an emulsion is formed nanoparticles 18 will congregate at the phase interface 14.

The nanoparticles 18 which stabilise the emulsion are preferably silica nanoparticles having a preferred average diameter of approximately 50 nm. However, it will be understood that the nanoparticles 18 may have an average diameter in the range 5 nm - 2000 nm and may be made from any suitable material, for example titania or latex, etc. Preferably, the ratio of nanoparticle size to capsule size is approximately, but not limited to, 1:15. In the preferred embodiment, the nanoparticles are Aerosil® silica nanoparticles obtained from Degussa AG. However, 80 nm titania nanoparticles and 100nm latex nanoparticles are also particularly suitable. The surfaces of nanoparticles 18 may be chemically or physically modified to hydrophobise nanoparticles 18. The resulting nanoparticle-encapsulated liquid droplet is referred to as a capsule 20.

It is an option that, prior to the addition of nanoparticles 18, a phospholipid monolayer, such as lecithin is used as a stabiliser to stabilise the emulsion (emulsifier 14 is shown in Figure 1). Lecithin is a fat emulsifier which may prevent droplets 10 from coalescing or ripening before nanoparticles 18 congregate. It will be understood by persons skilled in the art that other natural or synthetic stabilisers could be used to stabilise the emulsion.

Figure 2 is merely a schematic representation and therefore, the nanoparticles 18 are not drawn to scale with respect to droplets 10. It should also be clear that nanoparticles 18 form a coating over the surface of droplets 10 (phase interface 14).

Experiments investigating the formation of capsules 20 were performed with nanoparticles 18 having hydrophilic surfaces and other experiments with nanoparticles 18 having hydrophobic surfaces. Typical isotherms for hydrophilic silica particles adsorbing at the oil water interface 14 are shown in Figure 3 of International patent application No PCT/AU2006/000771 (WO 2006/130904). It is clear that salt addition dramatically increases nanoparticle adsorption. In the preferred embodiment, NaCl is used, however it will be understood by persons skilled in the art that any electrolyte may be used. It is believed that the free energy of nanoparticle adsorption increases significantly with salt addition due to a reduction in the range of particle-droplet and particle-particle lateral electrostatic repulsion.

It was observed that hydrophilic silica nanoparticles form densely packed monolayers with limited interfacial particle aggregation at salt concentrations greater than or equal to $10^{-3}$ M (0.01 M) NaCl. At concentrations of $10^{-2}$ and $10^{-1}$ M NaCl, adsorption amounts for hydrophilic nanoparticles 18 correspond to approximately 75% and just over 100% of an equivalent hexagonally close-packed monolayer of hard spheres respectively. The fractional surface coverage is an approximation calculated from the ratio of the adsorbed amount of nanoparticles 18 and the theoretical value for a hexagonally close-packed monolayer (i.e. 200 mg.m$^{-2}$ for 50 nm diameter nanoparticles).

Silica nanoparticles 18 can be modified to be hydrophobic. In the preferred embodiment, the surfaces of nanoparticles 18 are modified with organosilanes. The adsorption behaviour of hydrophobic nanoparticles 18 at the phase interface 14 is highly contrasting to that for hydrophilic nanoparticles. Salt addition still dramatically increases nanoparticle adsorption, for example, hydrophobic silica nanoparticles form rigid layers at greater than or equal to $10^{-4}$ M (0.001 M) NaCl, and thick interfacial walls at $10^{-2}$ M (0.1 M) M NaCl. However, attractive hydrophobic forces play a significant role and packing at the interface is not solely controlled by electrostatic repulsion. Surface coverage values increase to multiple layer values.

The coalescence behaviour of capsule 20 is dependent upon the hydrophobicity or hydrophilicity of nanoparticles 18, as well as the coverage of nanoparticles 18 at the emulsion droplet interface 14. At full or partial coverage of hydrophilic nanoparticles 18, capsules 20 still display enlargement behaviour, i.e. the diameter of the capsules increase during coalescence. In contrast, emulsion droplets coated by more than one layer of hydrophobic nanoparticles 18 (under conditions of coalescence), form stable flocculated networks rather than enlarged capsules. Experiments have revealed that in the wet phase, it is preferable that nanoparticles 18 have a hydrophobic surface which reduces the occurrence of capsule 20 coalescence.

Capsules 20 can have a liquid core or liquid medium 22 (the discontinuous phase) which may comprise an active substance 24. In the preferred embodiment, the liquid core 22 is a hydrophobic oil-based or lipidic medium and may contain a lipophilic active substance 24 therein. It is an option, however, that the liquid core 22 is hydrophilic (i.e. aqueous) and has a hydrophilic active substance 24 dissolved therein. In Figure 2, the cross-sectional schematic representation shows active substance 24. The active substance may be any substance which is required to be protected and/or delivered by capsule 20, e.g. a drug compound, a pesticide compound or a vitamin, etc. In the preferred embodiment, the active substance 24 is a drug compound. The active substance 24 may be wholly or partially soluble or dispersible within liquid core 22.

Capsules 20 show good shelf life properties and can be stored and/or transported for later use. In addition, capsules 20 may demonstrate reduced leaching of active substance 24 over time relative to prior formulations, and the nanoparticle 18 layer can be engineered so as to control active substance release within desired parameters. Depending upon the physical properties of the nanoparticles 18, an active substance 24 may continue to be released after many hours, or even days, have passed (i.e. sustained release), or in a short period of time (enhanced release).

Capsules 20 can be formed at relatively low temperatures, which is an advantage for temperature sensitive active substances such as biological active substances (e.g. peptides, proteins and nucleic acids).

It is an option that capsules 20 be coated with a layer that improves the interfacial properties of the capsules. For example, in drug delivery, capsules 20 may be further coated with a polymer layer around the periphery of capsule 20 to increase the bioadhesivity of the capsule to cells within the body. Such a polymer layer may be selected form the group consisting of methylcellulose, hydroxypropylcellulose, ethylcellulose, polyethyleneglycols, chitosan, guar gum, alginates, carbomers, eudragit and pemulen, etc. Other coatings around the capsule 20 which improve or modify the interfacial properties of the capsule may be used.

The quantity and properties of nanoparticles 18, added to the emulsion, is preferably selected so that capsules 20 can withstand a subsequent drying step. A delivery system which is dry and can be transported, stored and/or administered as a powder is an advantage in many industries, such as the pharmaceutical industry, since dry powder formulations usually have a higher active substance content compared with an aqueous formulation. This means that less volume of the delivery system is required for administration of an effective amount of active substance. The increase in active substance content in dry formulations is mainly due to the elimination of unnecessary liquids.

Figure 3 is a flow chart outlining a process for obtaining dried capsules. The first step 26 in the process is the formation of a two-phase liquid system having nanoparticles at the phase interface of an oil-in-water emulsion (the system depicted in Figure 2). The second step 28 involves removal of the continuous phase (water) by drying.

The first step 26 involves the selection of the nanoparticles' physical properties (i.e. hydrophilic or hydrophobic surface) and the amount of nanoparticles assembled at the interface (i.e. the fractional surface coverage of nanoparticles). The fractional surface coverage of nanoparticles can be controlled by varying the salt concentration and droplet/nanoparticle ratio. As described above, at high salt concentrations (e.g. $10^{-2}$ M NaCl), the adsorption of nanoparticles at the phase interface increases significantly.

The choice of whether to use hydrophilic or hydrophobic nanoparticles may be influenced by the intended use of the resulting capsules. For example, whether, in use, there will be dry or wet delivery of the capsules. Hydrophobic nanoparticles form a stable wet phase capsule with good protection of the active substance, however, preliminary experiments indicate that hydrophilic nanoparticles better stabilise capsules during a drying phase. Preliminary data also indicates that if the nanoparticles have a hydrophobic surface, then the capsules may be unstable during the drying step. This may be due to migration of the hydrophobic nanoparticles into the oil of the emulsion droplet, resulting in instability of the capsules.

It is an option therefore, which may prove beneficial by further experiment, that droplets are first coated with a hydrophobic layer of nanoparticles to create a stable wet phase. The resulting capsules can then be further coated by a hydrophilic layer of nanoparticles to stabilise the capsule during a drying phase.

In step 28, the emulsion is dried by rotary evaporation which removes the continuous phase by evaporation under reduced pressure. The resulting dried capsules can be collected in a suitable vessel. The emulsion can be dried by any suitable method, e.g. freeze drying, spray drying, fluidised bed procedures or pressure filtration combined with vacuum drying. However, it is believed that spray-drying of the capsules may offer better re-dispersibility of the capsules.

The table of Figure 4 shows that the ratio of the quantity of nanoparticles to the volume of oil droplets can be varied, as well as varying the salt concentration. For example, in row 1 of the table in Figure 4, 10 ml of an emulsion (prepared by mixing oil with water using a rotor-homogeniser) was mixed with 10 ml of a 1% wt aqueous dispersion of nanoparticles (dispersed by sonication). The overall volume of the mixture was 20 ml and the salt concentration of the mixture was $10^{-4}$ M NaCl. For further illustration, in row 7, 1 ml of an emulsion was mixed with 10 ml of a 1%wt aqueous dispersion of nanoparticles. The overall volume of the mixture was made up to 20 ml by the addition of 9 ml of water. The salt concentration of the mixture was $10^{-4}$ M NaCl.

Figure 4 shows that of the eighteen different variations in emulsion volume, amount of nanoparticles and salt concentration, twelve combinations formed capsules which maintained their integrity during a drying step. In the first six rows of the table, a dry powder of capsules could not be obtained due to degradation of capsules. Samples labelled A-L (in column 1) show the volume of emulsion to quantity of nanoparticles and corresponding salt concentrations which formed dry capsules.

Dried capsules have nanoparticles congregated at their surface, forming a phase boundary between liquid and the air. Once dried, it is an option that dried capsules are delivered in dry form. Dry formulations have increased active substance loading, thereby reducing the amount of formulation that is required. A further advantage is that the risk of microbial growth, which can cause serious infections or spoilage, is reduced in dry formulations compared with liquid formulation.

The capsules are prepared so as to remain stable and do not coalesce to form capsules with an increased diameter. The capsules therefore show good maintenance of the small capsule size as well as the release profile of the active substance contained within the capsule. The small size of the capsules both increases surface area and allows the capsules to be delivered to target areas which require a small capsule size, i.e. blood capillaries.

Alternatively, the dried capsules 20 can be re-dispersed (shown by step 30) in a liquid to re-form a stabilised emulsified product. An advantage of the dried capsules 20 is that they can re-disperse in a liquid to form an emulsion which is substantially identical in composition to the emulsion from which the capsules were dried.

The table of Figure 5 shows the results of re-dispersing the dried capsules labelled A-L in the table of Figure 4. The average capsule size prior to drying is shown along with the average capsule size following re-dispersion. The more closely the size values correlate, the better the stability of the capsule against enlargement due to coalescence.

It is clear that samples E, F, K and J showed the best re-dispersibility with the capsules in those samples maintaining a very small diameter as well as the percentage of capsules above 10 $\mu$m being desirably low.

It is clear from the description above, that the structure of the nanoparticle layer (i.e. coating) that forms around a droplet is dependent upon salt concentration and the nature of the silica nanoparticles, i.e. whether they are hydrophilic or hydrophobic. These layers are now related to drug release profiles from within the droplets.

In experiments, dibutylphthalate (DBP) was chosen as a model drug because it is a liquid that is poorly soluble but readily miscible with PDMS. Release profiles of DBP were determined from both bare droplets (i.e. droplets not coated with nanoparticles) and coated droplets (i.e. droplets coated with hydrophilic particles and from droplets coated with hydrophobic particles).

DBP was incorporated into the PDMS droplets during the synthesis step outlined in Example 3. A modification of the method reported by Obey, T.M. and Vincent, B., (1994), Journal of Colloid Interface Science, 163:454-463 and Goller, M.I. et al., (1997), Physiochemical and Engineering Aspects, 123-124 and 183-193 (without dialysis) was employed. PDMS droplets were prepared according to Example 3. A further batch of cross-linked PDMS droplets were prepared using the same procedure as for liquid droplets except that the mixtures of monomer and cross-linking trimer DEDMS:TEMS (tritethoxymethylsilane) at ratios 1: 0.1-1 were used instead of pure monomer. The cross-linking level of the droplets prepared ranged from 0, 10, 20, 30, 40 to 50%.

Bare and nanoparticle-coated droplet samples were prepared by mixing 10 ml of the prepared emulsions with 10 ml of sonicated MilliQ water and silica aqueous dispersions, respectively. Salt concentrations were adjusted from between $10^{-4}$ to $10^{-1}$ M NaCl in order to control the nanoparticle layer structure as described above.

DBP is a lipophilic molecule (water solubility 1mg/100ml at 20°C). When the drug is present at a concentration significantly below its solubility limit in water, the drug release from within the bare droplets is rapid and complete (Figure 6). The presence of hydrophilic silica and hydrophobic silica nanoparticles at low salt concentration (e.g. $10^{-4}$ M NaCl) does not significantly influence the rapid release of DBP. However, at higher salt concentrations ($10^{-3}$ and $10^{-2}$ M NaCl) and with hydrophobic silica nanoparticles, a rigid interfacial layer is created that significantly retards the release rate; the half release time is approximately 18 hours. The release rate is even more retarded in the presence of a thick interfacial particle wall prepared at $10^{-1}$ M NaCl. Thus, depending on salt concentration, hydrophobic silica nanoparticle coatings can provide a permeable or semi-permeable barrier.

From the release profiles for bare and coated droplets, it has been determined that interfacial transport is the rate limiting step in the release process of DBP from hydrophobic silica nanoparticle-coated droplets when rigid interfacial layers are present. The release rate of drug over long times, can be approximated by the equation:

$$M_t = 1/3 \; A \; c_0 \; r \; (1- \exp \; (-3\kappa\tau))$$

where A is the surface area of the sphere, $c_0$ is the initial concentration of the drug in the oil droplet and $\kappa$ is given by:

$$\kappa = k_1/D$$

$k_1$ is the interfacial rate constant; all remaining symbols have their previous meanings.

Since the initial amount of drug in the droplet is A $c_0$ r/3, this expression simplifies to:

$$M_t/M_0 = 1-\exp \; (-3 \; k_1t/ \; r^2)$$

and using the same linear transform as for the diffusion-limited case, the following equation A is obtained:

$$Ln \; (1- \; M_t/M_0) = -3 \; k_1t/r^2 \qquad\qquad \text{(equation A)}$$

A plot of Ln (1- $M_t/M_0$) against time will have a limiting slope at longer times of -3 $k_1/r^2$, enabling the interfacial transport rate constant of the drug, between the oil droplet and the release medium, to be found.

Figure 7 is a graph of Ln (1- $M_t/M_0$) against time. Correlation coefficients are > 0.96 and release rate constant were calculated to be 0.3 $nm^2$ $s^{-1}$ (at $10^{-3}$ and $10^{-2}$ M NaCl) and 0.05 $nm^2$ $s^{-1}$ (at $10^{-1}$ M NaCl). From literature such as Washington, C. and Evans, K., (1995), J. Contr. Rel., 33, 383-390, Barthel, H. et al. 2003, US Patent Publication No 2003/0175317, and Binks, P.B., (2002). Proceedings of 3rd World Congress on Emulsions, Lyon, CME, Paris, 1-10, it is possible to conclude that the nanoparticle coatings are a more significant barrier for molecular transport of DBP from emulsion droplets than are adsorbed polymers.

The activation energy for crossing the interfacial barrier was determined using an Arrhenius approach. Release profiles for droplets coated at $10^{-3}$ M NaCl and $10^{-1}$ M NaCl were determined at four temperatures: 22°C, 27°C, 32°C and 37°C. Kinetic rate constants were determined for each temperature from equation A above and from the plots Ln k vs. 1/T (Figure 8) the activation energies ($E_a$) were calculated:

$$\text{Slope} = -\frac{E_a}{R}$$

$$R = 8.31 \text{ J/Kmol}$$

$E_a$ values were calculated to be 580 and 630 kJmol$^{-1}$, for nanoparticle layer structures prepared at 10$^{-3}$ M NaCl and 10$^{-1}$ M NaCl, respectively. These values are significantly higher in comparison with $E_a$ values for small lipophilic molecules to pass polymeric type barriers (50 kJmol$^{-1}$) around oil droplets.

The linearity of the Arrhenius plots in Figure 8 can be attributed to insignificant changes in the interfacial nanoparticle layer structure during the release process. The attachment energy of small particles with intermediate contact angles (close to 90° at oil-water interfaces) has an order of magnitude of 104 kT, hence confirming irreversible attachment of the nanoparticles. Therefore, diffusion through the interfacial wall, not particle detachment, can be proposed as the drug release mechanism from these capsules. Kinetic parameters for the release process are presented in the table of Figure 9. These parameters reflect the correlation between interfacial layer structure and release profiles: there is no difference in the behaviour of the system at 10$^{-3}$ and 10$^{-2}$ M NaCl because of the similar interfacial rigid layer structure, whereas release is more retarded at 10$^{-1}$ M NaCl due to the presence of relatively thick interfacial particle walls.

In comparison with the sink conditions (i.e. wherein initial DBP concentration in emulsion (0.025 wt%) is significantly below (~15%) the solubility limit in water (0.28 mg/100ml maximal possible amount in dissolution medium), release profiles appeared different when the maximal drug concentration was slightly above the solubility limit (1.1 mg/100ml) (see Figure 10). When pure DBP oil phase is added in such concentration into the water dissolution medium, it takes approximately 20 hours to achieve the equilibrium solubility level; this is because the dissolution rate determines the release profile. However, the dissolution rate is increased when DBP is incorporated into PDMS emulsion droplets.

When the silica nanoparticles are present in DBP aqueous dispersion or at the surface of PDMS emulsion droplets containing DBP, the dissolution velocity and soluble drug fraction is dramatically increased. The effect is strongly dependent upon the nature of the nanoparticle coatings; it is only significant when permeable nanoparticle coatings are present at the surface of the droplets (i.e. hydrophilic silica coatings prepared at 10$^{-2}$ NaCl and hydrophobic silica coatings prepared at 10$^{-4}$ NaCl), whereas when there are relatively thick nanoparticle coatings around the droplets (eg hydrophobic nanoparticle coatings prepared at 10$^{-1}$ M NaCl), the increase in solubility of DBP is not as significant (this is probably due to the retarded diffusion across viscoelastic droplet interfaces (Figure 10)).

Similar trends of increased solubility rate occur when the total DBP concentration is well above the solubility limit (2.8 mg/100ml) (Figure 11). The observed increase in solubility of DBP is even more evident giving rise to supersaturated solutions. The intensity and duration of the "supersaturation" effect is much more pronounced for hydrophobic silica coated droplets. For hydrophilic particles, the peak in the soluble DBP concentration is at a maximum after 2 hours and then subsequently the soluble amount of DBP decreases within the next 6 hours and eventually reduces back to the solubility limit (Figure 11).

The "supersaturation" effect is more pronounced for hydrophobic silica nanoparticles, in terms of maximal solubility achieved as well as duration of the effect (i.e. after 10 hours, the amount in solution reduces to the normal solubility limit). It is speculated that this difference in the effect of hydrophilic and hydrophobic silica nanoparticles is a consequence of the amphiphilic nature of hydrophobic silica, which gives an opportunity for hydrophobic binding to DBP (higher amount of DBP adsorbed and higher amount released in solution). As in the previous case, the increase in solubility is negligible when thick interfacial walls of hydrophobic silica nanoparticles are present at the surface of the droplets (Figure 11).

Hydrophilic silica is an excellent additive to accelerate the dissolving process of actives that are difficult to dissolve, and thus it can improve the biological availability of a compound. Adsorbates of hydrophilic silica and poorly soluble drugs have been produced, so that non-polar solvents form loosely packed sorption layers which, upon contact with water, release sufficient quantities of active into the water so that supersaturated solutions are formed.

Considering that formation of saturated and supersaturated solutions occurs when either pure DBP or DBP within the droplets is mixed with silica nanoparticles, it is believed that DBP physisorps onto the silica adsorbed at the surface of the droplets, and upon dilution in water, DBP is desorbed and released in water.

Cross-linked droplets (40% cross-linked) were chosen for study due to the fact that DBP partitioning coefficients were the highest at this cross-linking level, i.e. entrapment of DBP was the highest. DBP release studies under sink conditions (0.025wt% DBP in the droplets) show that cross-linking of the droplets retards drug diffusion from the droplet (Figure 12). Hydrophilic silica nanoparticle-coated capsules (created at 10$^{-2}$ M NaCl) and permeable, hydrophobic silica nanoparticle-coated capsules (created at 10$^{-4}$ M NaCl) have no effect on drug dissolution as opposed to semi-permeable hydrophilic/hydrophobic silica nanoparticle coatings around capsules (created at 10$^{-3}$-10$^{-1}$ M NaCl).

The rate limiting step for drug release from bare cross-linked droplets is diffusion through the internal matrix, therefore the diffusion-limited model is applicable (Figure 12). Good linear fits were obtained for the first 120 minutes of release (Figure 13). Calculated diffusion coefficients are presented in the table of Figure 14 and are in agreement with typical values for drug diffusion in gels (e.g. 4.8 to 6.5 nm$^2$s$^{-1}$).

Diffusion is further sustained when hydrophobic silica nanoparticles are present as semi-permeable coatings. The release process reached equilibrium after approximately 2 hours. After 2 hours, 25 % of the amount of DBP loaded still remained in the droplets (for bare droplets and coated with permeable silica nanoparticle coatings at $10^{-4}$ M NaCl), (compare: 37% (for silica coating at $10^{-3}$ M NaCl) and 46% (for silica coating at $10^{-1}$ M NaCl)). Due to the presence of nanoparticles, the diffusion coefficients reduced to 3.2 and 2.4 $\pm$ 0.5 $nm^2s^{-1}$. The activation energy for drug diffusion from bare cross-linked droplets is 127 $\pm$ 15 $kJmol^{-1}$ and in the presence of a hydrophobic silica naoparticle coating, it becomes 155 and 177 $\pm$ 25 $kJmol^{-1}$ (Figure 14). Therefore, these represent major energy barriers for diffusion in the gel matrix of the droplets. In comparison, with liquid droplets, silica nanoparticle coatings are less effective diffusion barriers probably due to the lower particle penetration in the droplets, and consequently, lower interfacial viscosity.

For cross-linked droplets, when the DBP concentration is increased above the solubility limit (Figure 15), the dissolution profiles are clearly different than from liquid droplets, i.e. no supersaturated solutions are formed and an increase in solubility is only slightly pronounced for permeable, hydrophobic silica nanoparticle-coated capsules (created at $10^{-4}$ M NaCl). Calculated diffusion coefficients (Figure 16) (for the first 90 minutes) are 3.5$\pm$0.5 $nm^2s^{-1}$. The observed different behaviour of liquid and cross-linked droplets can be attributed to different release rate limiting steps, i.e. diffusion from the gel matrix is the rate-limiting step for cross-linked droplets and interfacial transport for the liquid droplets.

## EXAMPLES

### Example 1: Producing Nanoparticle-Stabilised Emulsion

#### a) Preparation and characterisation of emulsion stabilised by lecithin

Lecithin (0.6g) stabiliser was dissolved in oil (Miglyol 812™) (10g), and then added to water (total sample weight: 100g) under mixing using a rotor-stator homogeniser (11,000 rpm, 10 minutes, pH= 6.95 $\pm$ 0.2). After 24 hours, the emulsion was characterised in terms of size (laser diffraction Malvern Mastersizer) and zeta potential (PALS). The droplet size ranges from 0.20 - 0.86 $\mu$m.

For the inclusion of an active substance, the active substance may be added to the oil before or after the addition of the lecithin.

#### b) Preparation of nanoparticles

An aqueous dispersion of silica (Aerosil®) nanoparticles (1 wt%) was prepared by sonication over at least a one hour period. Figure 5 shows that the average size of the silica nanoparticles was approximately 50 nm.

#### c) Capsule formation

The emulsion formed in step (a) and the nanoparticle dispersion (b) were mixed together. The concentration of electrolyte of the two-phase liquid system was estimated to be within the range of about $10^{-4}$ M to $10^{-1}$ M (NaCl).

#### d) Adjusting electrolyte concentration

In this example, no additional electrolyte was added. At the estimated electrolyte concentration, it was anticipated that the formed capsules would comprise a layer of congregated nanoparticles that presents a semi-permeable barrier to the diffusion of any active substance included within the discontinuous phase.

The electrolyte concentration of the two-phase liquid system for formation of capsules can, however, be adjusted to vary the release characteristics of an active substance from the discontinuous phase.

### Example 2: Drying - Removal of Continuous Phase

The capsules formed in Example 1 were dried by rotary evaporation at 50°C, until the water phase was completely removed.

### Example 3: Preparation of Liquid PDMS droplets

Aqueous solutions containing 1% diethoxy-dimethyl-silane (DEDMS), which was previously mixed with 0, 0.025, 0.1 and 0.25 wt% DBP in a nitrogen gas atmosphere, and 0.1 % ammonia were sealed under nitrogen gas in a 250 ml reaction vessel, shaken vigorously for 30 seconds, and than tumbled at 30 rpm and 25 °C for 18 hours.

Drop size distributions were characterised by laser diffraction (Malvern Mastersizer X). Average drop sizes and size span [defined as (d(v,0.9) - d(v,0.1)) / d(v,0.5)] were ~ 2 $\mu$m and 0.56 for the liquid droplets, and 1.55 $\mu$m and 1.2 for the cross-linked droplets. The presence of DBP did not significantly change the drop size distribution.

The emulsion samples were considerably more mono-dispersed than typical o/w or w/o emulsions prepared by homogenisation. Electrophoretic mobilities and hence $\zeta$ potentials were determined using a combination of microelectrophoresis (Rank Bross, Mark H) and PALS; $\zeta$ potentials are not changed (within the experimental error) when DBP is present up to 0.25 wt%.

**Example 4: Preparation of Nanoparticle-Stabilised Emulsion of Vitamin A**

Retinol (Vitamin A alcohol) is an active substance of considerable interest to the pharmaceutical, nutritional and cosmetic industries. Formulating the substance has, however, been met with difficulties due to its sensitivity to oxidation (e.g. photo-oxidation upon exposure to light). In particular, Vitamin A alcohol is sensitive to auto-oxidation at the unsaturated side chain of the compound, resulting in the formation of decomposition products, isomerisation and polymerisation. As a result, auto-oxidation leads to reduced biological activity, and an increased risk of toxicity caused through generation of decomposition products. A nanoparticle stabilised emulsion of Vitamin A alcohol was produced in accordance to assess whether the present invention offered the possibility of providing a formulation showing enhanced stability of vitamin A alcohol, with a sustained rate of release.

**a) Preparation of Vitamin A-containing emulsion stabilised by lecithin**

Lecithin (0.6 g) stabiliser and all-*trans*-retinol (0.05 g) was dissolved in triglyceride oil (Miglyol 812™) (10 g), and then added to water (total sample weight: 100 g) under mixing using a rotor-stator homogeniser (11,000 rpm, 10 minutes, pH= 6.95 $\pm$ 0.2) or, alternatively, a high pressure homogeniser (5 mbars, 5 cycles). The concentration of electrolyte of the two-phase liquid system was estimated to be within the range of about 1 x $10^{-6}$ to 1 x $10^{-5}$ M (NaCl). No additional electrolyte was added.

**b) Preparation of Vitamin A-containing emulsion stabilised by oleylamine**

Oleylamine (1 g) stabiliser and all-*trans*-retinol (0.05 g) was dissolved in triglyceride oil (Miglyol 812™) (10 g), and then added to water (total sample weight: 100 g) under mixing using a rotor-stator homogeniser (11,000 rpm, 10 minutes, pH= 6.95 $\pm$ 0.2) or, alternatively, a high pressure homogeniser (5 mbars, 5 cycles). The concentration of electrolyte of the two-phase liquid system was estimated to be within the range of about 1 x $10^{-6}$ to 1 x $10^{-5}$ M (NaCl). No additional electrolyte was added.

**c) Preparation of nanoparticles**

An aqueous dispersion of fumed silica (Aerosil® 380) nanoparticles (1 wt%) (i.e. hydrophilic nanoparticles) was prepared by sonication over at least a one hour period.

**d) Capsule formation**

The emulsion formed in step (a) and step (b) was separately mixed with the nanoparticle dispersion of step (c).

**e) Alternative preparation**

Capsules may also be formed in an analogous manner wherein the nanoparticles are initially included in the triglyceride oil from which the emulsion is formed. For example, to prepare a lecithin-stabilised nanoparticle-coated vitamin A capsule similar to that described in a) above, all-*trans*-retinol (0.05 g) was dissolved in triglyceride oil (Miglyol 812™) (10 g) to which fumed silica (Aerosil® 380) nanoparticles (5 wt% in oil phase) and lecithin (0.6 g) stabiliser had previously been added, and then added to water (total sample weight: 100 g) before forming an emulsion using a rotor-stator homogeniser (11,000 rpm, 10 minutes, pH= 6.95 $\pm$ 0.2) or high pressure homogeniser.

**f) Capsule characteristics**

The nanoparticle-coated capsules formed were approximately 0.5 $\mu$m in diameter.

The capsules were assessed for stability of the retinol upon exposure to ultraviolet light. The results are shown in Figures 16 and 17. The positively charged nanoparticle-coated capsules (i.e. capsules stabilised with oleylamine) showed particularly good stability against UV exposure. While not wishing to be bound by theory, it is considered that the less pronounced results for the negatively charged nanoparticle-coated capsules (i.e. capsules stabilised with lecithin) may have been due to a stabilising effect conferred by the lecithin *per se* on the retinol.

The capsules were also assessed for *in vitro* drug (i.e. retinol) release. The analysis of the drug release profiles obtained (shown at Figures 18 and 19) showed that Higuchi's model is the most suitable for describing the release kinetics of the retinol:

$$Q_t = K_H \, t^{1/2}$$

Q: the amount of drug released in time t per unit area

$K_H$: Higuchi's rate constant;

and the calculation of diffusion rate constants (see Table 1) from the slope of the line in the plot of released amount of drug per unit area of the membrane versus $\sqrt{t}$ showed that the diffusion rate constant in the presence of silica nanoparticles decreased for both negatively and positively charged emulsions (i.e. the nanoparticle-coated capsules showed a sustained rate of retinol release).

**Table 1 Correlation of diffusion rate constant for the diffusion of drug from different formulations**

| Formulation | Rate Constant ($\mu g/cm^2/h^{1/2}$) | Correlation Coefficient |
|---|---|---|
| O/W | 0.88 | 0.9948 |
| L | 1.85 | 0.8690 |
| LSO | 1.10 | 0.9835 |
| LSA | 0.84 | 0.9598 |
| O | 1.07 | 0.9974 |
| OSO | 0.64 | 0.8871 |
| OSA | 0.92 | 0.9802 |

**Example 5: In vitro Release/Delivery from Nanoparticle-Stabilised Emulsion of Vitamin A**

**a) Lecithin stabilised formulations (negatively charged capsules)**

A study of the release profile of vitamin A from the lecithin-stabilised formulations described in Example 4 was undertaken using excised pig skin with Franz diffusion cells. The study was made in comparison with a lecithin-stabilised emulsion of vitamin A in the triglyceride oil. Briefly, the skin from the abdominal area of a large white pig was separated and after removal of hair and the underlying fat layer, was kept at -80° C until required. Skin samples were mounted to diffusion cells and 100 $\mu$l of the vitamin A formulation applied to achieve the thin layer on the skin sample surface. All experiments were carried out under occluded conditions.

At 6, 12 and 24 hours, skin samples were taken and extracted with acetone to determine the concentration of vitamin A retained in the whole skin. In addition, samples from receptor phase (ethanol/water 50/50) and skin surface were analysed with HPLC to quantify the penetrated ratio through the skin and the amount of drug remaining on the skin surface, respectively. The results are shown in Figures 20 and 21.

At all time points, the skin retention of vitamin A was increased significantly for the formulations compared to unencapsulated control emulsions stabilised with lecithin. The results were statistically analysed with T test and ANOVA test and significance is marked in Figure 20 with asterisks for P values less than 0.05.

The formulations are proposed for use in topical skin application (e.g. for cosmetic purposes) and, accordingly, the "target layer" for the delivery of the vitamin A is the upper layers of skin. Transport across the skin is undesirable in such application, and it simply leads to the "loss" of the active substance. Surprisingly, it was found that the amount of vitamin A detected in the receptor phase was negligible (Figure 21) for the formulations (i.e. less than 0.5%).

**b) Oleylamine stabilised formulations (positively charged capsules)**

A study of the release profile of vitamin A from the oleylamine-stabilised formulations described in Example 4 was also undertaken using excised pig skin with Franz diffusion cells. In this case, the study was made in comparison with a oleylamine-stabilised emulsion of vitamin A in the triglyceride oil.

The results obtained with these positively charged emulsions according to the present invention (see Figure 22) similarly showed enhancement in skin retention of vitamin A by nanoparticle encapsulation of the emulsion. Moreover, the oleyalmine-stabilised formulation generally showed higher skin retention and penetration (up to 1%) compared to the lecithin-stabilised formulations tested in a) above.

## Claims

1.  A method of producing a controlled release formulation for an active substance, said method comprising the steps of:

    (i) dispersing a discontinuous phase comprising 0.01 to 10wt% an active substance into a continuous phase in the presence of an emulsifier with an HLB value of less than 12 so as to form a two-phase liquid system comprising droplets of said discontinuous phase, each of said droplets having, at its surface, a phase interface; and
    (ii) allowing hydrophilic silica nanoparticles, provided to said two-phase liquid system to congregate at the phase interface to thereby coat said surface of the droplets in at least one layer of said nanoparticles, and wherein the ratio of nanoparticle size to the size of the nanoparticle-coated droplets does not exceed 1:15;

    wherein said two-phase liquid system is formed, or is otherwise adjusted, so as to have a concentration of sodium chloride (NaCl) electrolyte which controls the nanoparticle congregation of step (ii) by enhancing nanoparticle congregation such that the coating on said surface of the droplets provided by the at least one layer of said nanoparticles, presents a semi-permeable barrier to the active substance, thereby controlling release of the active substance.

2.  The method of claim 1, wherein the concentration of NaCl is within the range of $5 \times 10^{-4}$ to $5 \times 10^{-1}$ M preferably within the range of $1 \times 10^{-3}$ to $1 \times 10^{-1}$ M.

3.  The method of any one of claims 1 to 2, wherein the discontinuous phase is an oil-based or lipidic medium and the continuous phase is aqueous.

4.  The method of any one of claims 1 to 2, wherein the discontinuous phase is aqueous and each droplet is surrounded by a single or multiple lipid bi-layer to form a liposome, and the continuous phase is also aqueous.

5.  The method of any one of claims 1 to 4, wherein the active substance is selected from drug compounds and vitamins.

6.  The method of any one of claims 1 to 5, wherein the nanoparticles have an average diameter of 20 - 80 nm.

7.  The method of any one of claims 1 to 6, wherein the nanoparticles are provided to the two-phase liquid system by inclusion in the discontinuous phase.

8.  The method of any one of claims 1 to 7, wherein the emulsifier is selected from the group consisting of lecithin, oleylamine, sodium deoxycholate, 1,2-distearyl-sn-glycero-3- phosphatidyl ethanolamine-N, stearylamine, amino acids and I,2-dioleoyl-3-trimethylammonium-propane.

9.  The method of any one of claims 1 to 8, further comprising the step of:

    (iii) drying the produced formulation.

## Patentansprüche

1.  Verfahren zur Herstellung einer Formulierung mit kontrollierter Freisetzung für einen Wirkstoff, wobei das Verfahren die folgenden Schritte umfasst:

    (i) Dispergieren einer diskontinuierlichen Phase, umfassend 0,01 bis 10 Gew.-% eines Wirkstoffs, in eine kontinuierliche Phase in der Gegenwart eines Emulgators mit einem HLB-Wert von weniger als 12, um ein zweiphasiges Flüssigkeitssystem zu bilden, das Tröpfchen der diskontinuierlichen Phase umfasst, wobei jedes der Tröpfchen auf seiner Oberfläche eine Phasengrenze aufweist; und

(ii) Ermöglichen, dass hydrophile Siliziumdioxid-Nanopartikel, die dem zweiphasigen Flüssigkeitssystem bereitgestellt werden, sich an der Phasengrenze ansammeln, um dadurch die Oberfläche der Tröpfchen mit mindestens einer Schicht der Nanopartikel zu beschichten, und wobei das Verhältnis von Nanopartikelgröße zur Größe der mit Nanopartikeln beschichteten Tröpfchen 1:15 nicht überschreitet;

wobei das zweiphasige Flüssigkeitssystem gebildet oder anderweitig angepasst wird, um eine Natriumchlorid (NaCl)-Elektrolyt-Konzentration zu haben, die die Nanopartikelansammlung von Schritt (ii) steuert, indem die Nanopartikelansammlung so verstärkt wird, dass die Beschichtung auf der Oberfläche der Tröpfchen, die von mindestens einer Schicht der Nanopartikel bereitgestellt wird, eine halbdurchlässige Barriere für den Wirkstoff präsentiert, wodurch die Freisetzung des Wirkstoffs gesteuert wird.

2. Verfahren nach Anspruch 1, wobei die NaCl-Konzentration im Bereich von $5 \times 10^{-4}$ bis $5 \times 10^{-1}$ M, vorzugsweise im Bereich von $1 \times 10^{-3}$ bis $1 \times 10^{-1}$ M liegt.

3. Verfahren nach einem der Ansprüche 1 bis 2, wobei die diskontinuierliche Phase ein ölbasiertes oder lipidisches Medium ist und die kontinuierliche Phase wässrig ist.

4. Verfahren nach einem der Ansprüche 1 bis 2, wobei die diskontinuierliche Phase wässrig ist und jedes Tröpfchen von einer einzelnen oder mehrfachen Lipid-Doppelschicht umgeben ist, um ein Liposom zu bilden, und wobei die kontinuierliche ebenfalls Phase wässrig ist.

5. Verfahren nach einem der Ansprüche 1 bis 4, wobei der Wirkstoff aus Arzneimittel-Verbindungen und Vitaminen ausgewählt ist.

6. Verfahren nach einem der Ansprüche 1 bis 5, wobei die Nanopartikel einen durchschnittlichen Durchmesser von 20 - 80 nm haben.

7. Verfahren nach einem der Ansprüche 1 bis 6, wobei die die Nanopartikel dem zweiphasigen Flüssigkeitssystem durch Einschluss in die diskontinuierliche Phase bereitgestellt werden.

8. Verfahren nach einem der Ansprüche 1 bis 7, wobei der Emulgator aus der Gruppe bestehend aus Lecithin, Oleylamin, Natriumdeoxycholat, 1,2-Distearyl-sn-glycero-3-phosphatidylethanolamin-N, Stearylamin, Aminosäuren und 1,2-Dioleoyl-3-trimethylammoniumpropan ausgewählt ist.

9. Verfahren nach einem der Ansprüche 1 bis 8, ferner umfassend den folgenden Schritt:

(iii) Trocknen der hergestellten Formulierung.

**Revendications**

1. Procédé de production d'une formulation à libération contrôlée d'une substance active, ledit procédé comprenant les étapes qui consistent à :

(i) disperser une phase discontinue comprenant de 0,01 à 10% en poids d'une substance active dans une phase continue en la présence d'un émulsifiant ayant une valeur de l'équilibre hydrophile/lipophile (HLB) inférieure à 12 pour former un système liquide à deux phases comprenant des gouttelettes de ladite phase discontinue, chacune desdites gouttelettes ayant, à sa surface, une interface de phases ; et
(ii) laisser des nanoparticules de silice hydrophile fournies au dit système liquide à deux phases se rassembler à l'interface de phases pour ainsi enrober ladite surface des gouttelettes avec au moins une couche desdites nanoparticules, le rapport entre la taille des nanoparticules et la taille des gouttelettes enrobées par des nanoparticules ne dépassant pas 1:15 ;

où ledit système liquide à deux phases est formé, ou sinon ajusté, de manière à présenter une concentration en l'électrolyte qu'est le chlorure de sodium (NaCl) qui contrôle le rassemblement des nanoparticules à l'étape (ii) en augmentant le rassemblement des nanoparticules de sorte que l'enrobage de ladite surface des gouttelettes produit par la au moins une couche desdites nanoparticules constitue une barrière semi-perméable à la substance active, contrôlant ainsi la libération de la substance active.

2. Procédé de la revendication 1, où la concentration en NaCl est dans la plage qui va de $5 \times 10^{-4}$ à $5 \times 10^{-1}$ M, de préférence dans la plage qui va de $1 \times 10^{-3}$ à $1 \times 10^{-1}$ M.

3. Procédé de l'une quelconque des revendications 1 à 2, où la phase discontinue est un milieu à base d'huile ou lipidique et la phase continue est aqueuse.

4. Procédé de l'une quelconque des revendications 1 à 2, où la phase discontinue est aqueuse, chaque gouttelette est entourée par des bicouches lipidiques uniques ou multiples pour former un liposome et la phase continue est également aqueuse.

5. Procédé de l'une quelconque des revendications 1 à 4, où la substance active est sélectionnée parmi des composés médicamenteux et des vitamines.

6. Procédé de l'une quelconque des revendications 1 à 5, où les nanoparticules ont un diamètre moyen de 20 - 80 nm.

7. Procédé de l'une quelconque des revendications 1 à 6, où les nanoparticules sont fournies au système liquide à deux phases par inclusion dans la phase discontinue.

8. Procédé de l'une quelconque des revendications 1 à 7, où l'émulsifiant est sélectionné dans le groupe consistant en les suivants : lécithine, oléylamine, désoxycholate de sodium, 1,2-distéaroyl-sn-glycéro-3-phosphatidyléthanolamine, N-stéarylamine, acides aminés et 1,2-dioléoyl-3- triméthylammonium-propane.

9. Procédé de l'une quelconque des revendications 1 à 8, qui comprend en outre l'étape de :

   (iii) séchage de la formulation obtenue.

**Fig 1**

*Fig 2a*

*Fig 2b*

*Fig 2c*

```
┌─────────────────────────────────┐
│    Formation of particle        │
│     stabilised emulsion         │
│ - selection of nanoparticle properties │  ╲_ 26
│ - selection of amount of nanoparticles │
└─────────────────────────────────┘
                │
                ▼
┌─────────────────────────────────┐
│      Drying (removal of         │
│      continuous phase)          │  ╲_ 28
└─────────────────────────────────┘
        │                    │
        ▼                    ▼
┌──────────────────┐  ┌──────────────────┐
│ Redispesion to reform │  │   Delivery of    │
│     emulsion     │  │   dry capsules   │
└──────────────────┘  └──────────────────┘
   30 ╲                           ╲_ 32
```

Fig 3

| Row No. | Experiment label | Volume of emulsion | Volume of nanoparticle 1wt% dispersion | M NaCl | Overall volume of the mixture |
|---|---|---|---|---|---|
| 1 | Powder cannot be obtained | 10 ml | 10 ml | $10^{-4}$ | 20 ml |
| 2 | Powder cannot be obtained | 10 ml | 10 ml | $10^{-2}$ | 20 ml |
| 3 | Powder cannot be obtained | 10 ml | 5 ml | $10^{-4}$ | 20 ml |
| 4 | Powder cannot be obtained | 10 ml | 5 ml | $10^{-2}$ | 20 ml |
| 5 | Powder cannot be obtained | 10 ml | 1 ml | $10^{-4}$ | 20 ml |
| 6 | Powder cannot be obtained | 10 ml | 1 ml | $10^{-2}$ | 20 ml |
| 7 | A | 1 ml | 10 ml | $10^{-4}$ | 20 ml |
| 8 | B | 1 ml | 10 ml | $10^{-2}$ | 20 ml |
| 9 | C | 1 ml | 10 ml | $10^{-1}$ | 20 ml |
| 10 | D | 1 ml | 10 ml | $10^{-4}$ | 11 ml |
| 11 | E | 1 ml | 5 ml | $10^{-4}$ | 20 ml |
| 12 | F | 1 ml | 5 ml | $10^{-2}$ | 20 ml |
| 13 | G | 1 ml | 5 ml | $10^{-1}$ | 20 ml |
| 14 | H | 1 ml | 5 ml | $10^{-4}$ | 6 ml |
| 15 | I | 1 ml | 5 ml | $10^{-2}$ | 6 ml |
| 16 | J | 1 ml | 5 ml | $10^{-4}$ | 10 ml |
| 17 | K | 1 ml | 5 ml | $10^{-2}$ | 10 ml |
| 18 | L | 1 ml | 5 ml | $10^{-1}$ | 10 ml |

Fig 4

| Experiment label | Average capsule size before drying (μm) | Average capsule size once redispersed (μm) | Vol% of capsules above 10 μm in size following redispersion | Redispersibility comment |
|---|---|---|---|---|
| A | 1.04 | 1.27 | 1.75 | good |
| B | 1.82 | 1.99 | 4.59 | good |
| C | 8.93 | 22.04 | 18.89 | poor |
| D | 13.67 | 28.89 | 24 | very poor |
| E | 0.76 | 1.04 | 0.2 | very good |
| F | 0.94 | 1.55 | 0.5 | very good |
| G | 12.99 | 56.6 | 87 | very poor |
| H | 26.75 | 47.3 | 89 | very poor |
| I | 5.87 | 30.05 | 30.8 | very poor |
| J | 1.52 | 2.51 | no | very good |
| K | 0.88 | 2.15 | no | very good |
| L | 1.36 | 5.23 | 1.5 | good |

Fig 5

Fig 6

Fig 7

Fig 8

Fig 9

Parameters for DBP release process from hydrophobic nanoparticle coated PDMS droplets prepared at different salt concentrations (0.025 wt% DBP in emulsion)

| Sample (PDMS droplets) | release 295.15K | rate 300.15K | constant 305.15K | $(nm^2 s^{-1})*$ 310.15K | $E_a$ $(kJmol^{-1})$ |
|---|---|---|---|---|---|
| coated at $10^{-3}$ NaCl | $10^{-3}$ | $10^{-2}$ | $10^{-2}$ | 0.3 | 580±65 |
| coated at $10^{-2}$ NaCl | $10^{-3}$ | $10^{-2}$ | $10^{-2}$ | 0.3 | 580±65 |
| coated at $10^{-1}$ NaCl | $2 \times 10^{-7}$ | $10^{-5}$ | $10^{-3}$ | $5 \times 10^{-2}$ | 630±75 |

* Determined from eq. 7.7; coefficient of variation maximally 12%

Fig 10

Fig 11

Fig 12

Fig 13

Parameters for drug release from cross-linked PDMS droplets ($\phi$=0.01) containing 0.025 wt% DBP, 900 ml MQ water as dissolution medium

| | Diffusion coefficient $(nm^2s^{-1})$ Temperature (K) | | | | Activation energy for drug diffusion $(kJmol^{-1})^*$ |
|---|---|---|---|---|---|
| | 310.15 | 305.15 | 300.15 | 295.15 | |
| Bare PDMS | 4.8±1.1 | 4.7±1.1 | 4.6±1.1 | 4.5±1.1 | 127±15 |
| hydrophobic silica coated($10^{-4}$ M | 5.5±1.1 | 5.4±1.1 | 5.3±1.1 | 5.2±1.1 | 110±15 |
| hydrophobic silica coated ($10^{-3}$ M NaCl) | 3.2±0.5 | 3.0±0.5 | 2.75±0.5 | 2.5±0.5 | 155 ± 25 |
| hydrophobic silica coated ($10^{-1}$ M NaCl) | 2.4±0.5 | 2.05±0.5 | 1.4±0.5 | 1.1±0.5 | 177± 25 |

*calculated from Arrhenius plot (Figure 7.12)

Fig 14

Fig 15

Fig 16

Fig 17

Fig 18

Fig 19

Fig 20

Fig 21

Fig 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006130904 A
- WO 2007115134 A
- AU 2006000771 W
- US 20030175317 A, Barthel, H.

**Non-patent literature cited in the description**

- **OBEY, T.M. ; VINCENT, B.** *Journal of Colloid Interface Science,* 1994, vol. 163, 454-463
- **GOLLER, M.I. et al.** *Physiochemical and Engineering Aspects,* 1997, 123-124, 183-193
- **WASHINGTON, C. ; EVANS, K.** *J. Contr. Rel.,* 1995, vol. 33, 383-390
- **BINKS, P.B.** *Proceedings of 3rd World Congress on Emulsions,* 2002, 1-10